(19) Europäisches Patentamt
European Patent Office
Office européen des brevets

(11) Veröffentlichungsnummer: **0 353 580 B1**

(12) **EUROPÄISCHE PATENTSCHRIFT**

(45) Veröffentlichungstag der Patentschrift: **29.09.93**

(51) Int. Cl.5: **C07C 227/08**, C07C 231/12, C07C 229/12, C07C 233/34

(21) Anmeldenummer: **89113570.9**

(22) Anmeldetag: **24.07.89**

(54) **Verfahren zur Herstellung von konzentrierten fliessfähigen wässrigen Lösungen von Betainen.**

(30) Priorität: **05.08.88 DE 3826654**

(43) Veröffentlichungstag der Anmeldung:
**07.02.90 Patentblatt 90/06**

(45) Bekanntmachung des Hinweises auf die Patenterteilung:
**29.09.93 Patentblatt 93/39**

(84) Benannte Vertragsstaaten:
**CH DE ES FR GB IT LI SE**

(56) Entgegenhaltungen:
**EP-A- 0 020 907**
**EP-A- 0 243 619**
**EP-A- 0 302 329**
**US-A- 3 225 074**
**US-A- 4 243 549**

(73) Patentinhaber: **Th. Goldschmidt AG**
**Goldschmidtstrasse 100**
**D-45127 Essen(DE)**

(72) Erfinder: **Bade, Volkbert, Dr.**
**Graffweg 38 b**
**D-4300 Essen 14(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein Verfahren zur Herstellung von konzentrierten fließfähigen wäßrigen, gegebenenfalls niedere aliphatische Alkohole, vorzugsweise Ethanol, enthaltenden Lösungen von Betainen der allgemeinen Formel

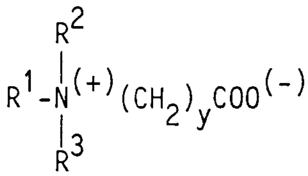

I

| | |
|---|---|
| $R^1$ | Alkylrest mit 6 bis 18 Kohlenstoffatomen oder der Rest $R^4CONH(CH_2)_x$-, wobei $R^4$ ein von einer Fettsäure mit 6 bis 18 Kohlenstoffatomen abgeleiteter Alkylrest und x = 2 oder 3 ist, |
| $R^2$, $R^3$ | gleich oder ungleich, Alkylrest mit 1 bis 4 Kohlenstoffatomen, |
| x | 2 oder 3, |
| y | 1,2 oder 3, |

durch Quaternierung eines tertiären Amins der allgemeinen Formel

$R^1$-$NR^2R^3$ II

mit ω-Halogenalkylcarbonsäuren $X(CH_2)_yCOOY$ oder deren Salzen (X = Halogenrest, Y = Wasserstoff-, Alkali- oder Ammoniumion) in wäßriger oder wäßrig-alkoholischer Lösung bei erhöhten Temperaturen.

Die Erfindung betrifft insbesondere die Herstellung von fließfähigen wäßrigen oder wäßrig-ethanolischen Betainlösungen mit möglichst hohem Betaingehalt, welche homogen sind und eine hohe Kältestabilität aufweisen.

Betaine der Formel I haben in den vergangenen Jahren zunehmende Bedeutung zur Herstellung von Körperreinigungsmitteln erlangt. Sie verbinden ausgezeichnete Reinigungseigenschaften mit guter Hautverträglichkeit. Die Betaine bilden in wäßriger Lösung einen stabilen dichten Schaum aus, der auch in Gegenwart von Seife nicht zusammenfällt.

Die Herstellung dieser Betaine ist in vielen Patentschriften beschrieben, von denen die US-PS 3 225 074 stellvertretend genannt wird. Im allgemeinen setzt man dabei das entsprechende tertiäre Amin der allgemeinen Formel II mit dem Alkalisalz einer ω-Halogencarbonsäure, in der Regel dem Natriumsalz der Chloressigsäure, um. Die Reaktion läuft vorzugsweise in wäßrigem Medium ab. Das bei der Reaktion entstandene Alkalichlorid verbleibt in der Lösung und wird nicht entfernt.

Die Betaine des Standes der Technik werden meist in Form ihrer 30 gew.-%igen wäßrigen Lösungen in den Handel gebracht. Es hat nicht an Versuchen gefehlt, höher konzentrierte Lösungen der Betaine herzustellen, um den Transport und die Lagerung der Betainlösungen zu verbilligen. Entzieht man jedoch den nach dem Stand der Technik erhältlichen Betainlösungen Wasser, steigt die Viskosität der Lösungen schnell an. Lösungen von Betainen auf Basis gehärteter Kokosfettsäure werden bei einem Gehalt von etwa 35 bis 37 Gew.-% Betain pastös und verfestigen sich bei weiterer Entwässerung zunehmend. Die Konzentration, von der ab Betainlösungen nicht mehr fließfähig sind, wird durch die Kohlenstoffanzahl des Restes $R^1$ bzw. $R^4$ beeinflußt. Je größer die Kettenlänge des Alkylrestes bzw. der Fettsäure oder des zur Herstellung des Betains verwendeten Fettsäuregemisches ist, um so stärker erhöht sich die Viskosität der Betainlösungen bei steigender Konzentration.

Es ist dem Fachmann zwar bekannt, daß mit steigender Konzentration einer wäßrigen Tensidlösung auch die Viskosität ansteigt. Häufig zeigt sich jedoch bei Überschreiten einer Konzentration von etwa 60 bis 70 Gew.-%, daß die Viskosität bei weiterer Konzentrationserhöhung auf ein Minimum abfällt, um dann erneut stark anzusteigen. Zur Erklärung dieser Viskositätsanomalie nimmt man an, daß sich in der Lösung eine G-Phase mit lamellarer Struktur ausbildet (Soap, Perfumery, Cosmetics, 1982, 507 bis 509). Ein solches Verhalten konnte aber bei Betainen nicht beobachtet werden. Auch bei weiterem Wasserentzug verflüssigen sich die verfestigten Produkte nicht mehr.

In der DE-PS 36 13 944 ist ein Verfahren zur Herstellung einer fließ- und pumpfähigen, mindestens 70 Gew.-% Betain der Formel I enthaltenden Lösung beschrieben, bei dem man

a) als Salz der Halogencarbonsäure das Ammoniumsalz einsetzt,

b) die Quaternierung in einem organischen polaren Lösungsmittel, welches höchstens 20 Gew.-% Wasser enthalten darf, durchführt,

c) nach der Quaternierung das gegebenenfalls enthaltene Wasser azeotrop abdestilliert und das ausgefallene Ammoniumhalogenid abtrennt, danach

d) das Lösungsmittel ganz oder teilweise abdestilliert und

e) vor, gleichzeitig oder nach der Destillation die gewünschte Konzentration des Betains in dem anwendungstechnisch gewünschten Lösungsmittel oder Lösungsmittelgemisch einstellt.

Bei diesem Verfahren muß somit die Quaternierung in einem organischen polaren Lösungsmittel, welches höchstens 20 Gew.-% Wasser enthalten darf, durchgeführt werden. Bei dem Schritt e) wird ein weiteres Lösungsmittel, vorzugsweise ein aliphatisches Diol, zur Einstellung der gewünschten Konzentration der Betainlösung zugesetzt. Die Betainlösungen haben dabei vorzugsweise folgende Zusammensetzung:

70 bis 90 Gew.-% Betain
2 bis 15 Gew.-% aliphatisches Diol
0,5 bis 10 Gew.-% Ethanol
0 bis 5 Gew.-% Wasser

Es besteht jedoch nach wie vor der Bedarf, möglichst konzentrierte wäßrige Lösungen von Betainen der Formel I herzustellen, welche trotz ihrer vergleichsweise hohen Konzentration fließ- und pumpfähig und vorzugsweise frei von Lösungsmitteln, außer Wasser und gegebenenfalls niederen Alkoholen, wie Methanol, Ethanol, Propanol oder Isopropanol, sind. Die niedrige Viskosität der wäßrigen Lösungen ist insbesondere erforderlich, um die Betainlösungen bei der Weiterverarbeitung fördern und dosieren zu können. Darüber hinaus besteht ein erhebliches wirtschaftliches Interesse, die Verpackungs-, Transport- und Lagerkosten sowie den Handhabungsaufwand zu erniedrigen. Dabei ist es von besonderer Wichtigkeit, daß die konzentrierten wäßrigen oder wäßrig-alkoholischen Lösungen beim Verdünnen ohne Gelbildung in Wasser verteilt werden können.

Ein weiterer Vorteil ist, daß die konzentrierten Tensidlösungen von sich aus vor einem Befall mit Mikroorganismen geschützt sind und deshalb nicht mehr konserviert werden müssen. Dies allein ist schon ein entscheidender Vorteil, da es auf diese Weise möglich ist, dem Verwender die freie Wahl eines Konservierungsstoffes in seinem verdünnten Endprodukt zu überlassen.

Überraschenderweise wurde nun gefunden, daß man fließfähige Lösungen von Betainen der allgemeinen Formel I erfindungsgemäß dadurch herstellen kann, daß man dem Reaktionsgemisch vor oder während der Quaternierungsreaktion oder der erhaltenen Lösung des Betains nichtionogene, wasserlösliche Tenside in solchen Mengen zusetzt, daß die fertige Lösung 3 bis 20 Gew.-%, vorzugsweise 3 bis 15 Gew.-%, nichtionogene Tenside enthält.

Durch den erfindungsgemäßen Zusatz der nichtionogenen Tenside wird die Betainlösung bezüglich ihrer Verarbeitungseigenschaften mehrfach verbessert:

a) Die Lösungen weisen eine derart erniedrigte Viskosität auf, daß eine einfache Handhabung der Lösungen möglich ist. Die Lösungen sind ohne weiteres fließfähig. Sie lassen sich ohne Schwierigkeiten fördern und dosieren.

b) Die Lösungen lassen sich in Wasser leicht verdünnen.

c) Die Lösungen sind in weiten Konzentrationsbereichen homogen und zeigen keine Entmischung.

d) Die Lösungen sind kältestabil und trüben bis zu Temperaturen von etwa 0 °C nicht ein.

Als wasserlösliche, nichtionogene Tenside sind die Polyoxyethylenester von Fettsäuren oder die Polyoxyethylenether von partiellen Fettsäureestern mehrwertiger Alkohole, wie Glycerin oder Sorbit, bevorzugt. Die zur Herstellung dieser nichtionogenen Tenside verwendeten Fettsäuren sind vorzugsweise die in der Natur vorkommenden Fettsäuren mit 8 bis 18 Kohlenstoffatomen. Sie können gesättigt oder ungesättigt sein und gegebenenfalls Hydroxylgruppen, wie die Rizinusfettsäure, aufweisen. Beispiele solcher Fettsäuren sind die Laurin-, Stearin-, Öl- und Rizinusfettsäure sowie aus natürlichen Fetten gewonnene Fettsäuregemische.

Geeignet, wenn auch nicht bevorzugt, sind die Polyoxyethylenether von Fettalkoholen mit 8 bis 18 Kohlenstoffatomen, wobei der Fettalkohol gesättigt oder ungesättigt, substituiert oder unsubstituiert sein kann. Beispiele solcher Fettalkohole sind der Lauryl-, Stearyl- und Oleylalkohol.

Vorzugsweise soll der HLB-Wert dieser nichtionogenen Tenside in einem Bereich von 14 bis < 20 liegen.

Hat $R^1$ die Bedeutung eines Alkylrestes, ist der bevorzugte HLB-Bereich 14 bis 18. Hat $R^1$ die Bedeutung des Restes $R^4CONH(CH_2)_x$, ist der bevorzugte HLB-Bereich 16 bis < 20.

Besonders geeignete und bevorzugte nichtionogene Tenside sind die Polyoxyethylenester von Fettsäuren oder Polyoxyethylenether von partiellen Fettsäureestern des Glycerins oder des Sorbits, mit einem durchschnittlichen Gehalt von 10 bis 250 Oxyethyleneinheiten. Für Betaine, deren Rest $R^1$ die Bedeutung

$R^4CONH-(CH_2)_x$- hat, können als Beispiele genannt werden:
Polyoxyethylenether von Sorbitanmonolaurinsäureester mit 120 Oxyethyleneinheiten,
Polyoxyethylenether von Sorbitanmonoölsäureester mit 200 Oxyethyleneinheiten,
Polyoxyethylenether von Sorbitanmonostearinsäureester mit 80 Oxyethyleneinheiten,
Polyoxyethylenester von Rizinusfettsäure mit 120 Oxyethyleneinheiten,
Polyoxyethylenester von Kokosölfettsäure mit 80 Oxyethyleneinheiten.

Ist $R^1$ dagegen ein Alkylrest, sind die vorgenannten Verbindungen besser geeignet, wenn ihr Gehalt an Oxyethyleneinheiten deutlich verringert ist. So ist z.B. ein Sorbitanmonolaurat mit 20 Oxyethyleneinheiten besonders geeignet.

Die Bildung niedrigviskoser hochkonzentrierter Betainlösungen wird noch dadurch unterstützt, daß man den Salzgehalt der Betainlösung möglichst niedrig hält. Eine weitere Ausgestaltung des erfindungsgemäßen Verfahrens ist deshalb dadurch gekennzeichnet, daß man die Quaternierungsreaktion in wäßriger, mindestens 20 Gew.-% Ethanol enthaltender Lösung durchführt, das ausgefallene Salz nach der Quaternierungsreaktion abfiltriert und die gewünschte Konzentration des Betains in der Lösung durch Abdestillieren des Wassers bzw. des Wasser/Ethanolgemisches einstellt. Es ist natürlich auch möglich, die durch Abdestillation des Wassers oder Wasser/Ethanolgemisches erhaltene hochkonzentrierte Lösung mit Wasser oder Wasser/Ethanolmischungen auf die gewünschte Konzentration einzustellen. Der als Lösungsmittel verwendete Ethanol kann bei dem Herstellungsverfahren im Kreislauf geführt werden, wobei die Konzentration des Ethanols im Wasser der des azeotrop siedenden Wasser/Ethanolgemisches entsprechen kann.

Es ist bekannt, daß die Viskosität wäßriger Lösungen anionaktiver Tenside durch Zusatz von amphoteren Tensiden erniedrigt werden kann. Zu diesem Stand der Technik wird die US-PS 4 243 549 genannt. In der Patentschrift wird eine wäßrige, gießfähige Tensidzubereitung beschrieben, die im wesentlichen aus Wasser und einer Wirksubstanzmischung besteht, wobei die Zubereitung auf eine Tensidkonzentration von 5 bis 30 Gew.-% verdünnbar sein soll. Die Wirksubstanzmischung besteht zu jeweils wenigstens 10 Gew.-% aus einem amphoteren und einem anionaktiven Tensid. Diesem Gemisch kann zusätzlich noch ein nichtionogenes Tensid zugegeben werden. Die Anwesenheit des amphoteren Tensids ist aber zwingend vorgeschrieben. Es war deshalb überraschend, daß wäßrige Lösungen von Betainen allein durch Zusatz geringer Mengen nichtionogener Tenside verflüssigt werden können.

Die Viskosität der wäßrigen bzw. wäßrig-alkoholischen Betainlösungen hängt natürlich auch von der Art des Restes $R^1$ in der Formel I ab. Im allgemeinen gilt, daß sich die Viskosität der Lösungen bei gleicher Konzentration mit steigender Kohlenstoffzahl des Restes $R^1$ erhöht. Bevorzugt sind deshalb als Reste $R^1$ Alkylreste mit einer mittleren Kohlenstoffzahl von 8 bis 14 bzw. $R^4CONH-(CH_2)_x$-Reste, deren Rest $R^4$ eine mittlere Kohlenstoffzahl von 7 bis 13 aufweist. In der folgenden Tabelle I wird gezeigt, welchen Einfluß die Kohlenstoffanzahl des Restes $R^4$ auf die Viskosität der erfindungsgemäß hergestellten Lösungen hat. Es wird dabei die Konzentration einer erfindungsgemäß hergestellten Betainlösung ermittelt, die eine Viskosität von ca. 20 000 mPa.s bei 25 °C aufweist, also noch gerade fließfähig ist. Dieser Bereich kann als Fließgrenze bezeichnet werden.

Tabelle I

| $R^4$ abgeleitet von | Konzentration bei ca. 20 000 mPa.s | Konzentration in Gew.-% nichtionogenes Tensid* | KCl | Ethanol | $H_2O$ |
|---|---|---|---|---|---|
| $C_8$-Fettsäure | 75 | 7,6 | 4,1 | 6 | 6,3 |
| $C_{10}$-Fettsäure | 69 | 7,0 | 4,6 | 6 | 12,4 |
| $C_{12}$-Fettsäure | 64 | 6,5 | 5,0 | 6 | 17,5 |
| $C_{14}$-Fettsäure | 62 | 6,2 | 5,2 | 6 | 19,6 |
| Kokosfettsäure, gehärtet | 65 | 6,5 | 5,5 | 6 | 16,1 |
| Kokos-/Palmkernfettsäure, gehärtet Gew.-Verhältnis 1 : 1 | 65 | 6,5 | 5,5 | 6 | 16,0 |

* Das nichtionogene Tensid ist ein Polyoxyethylenether von Glycerinmonorizinusfettsäureester mit 15 Oxyethyleneinheiten

Die Viskosität wird auch durch den Gehalt der erfindungsgemäß hergestellten Lösung an nichtionogenem Tensid bestimmt. In der folgenden Tabelle II ist die Abhängigkeit der Viskosität von der Konzentration des nichtionogenen Tensids in der Betainlösung gezeigt. Als Betain wird eine Verbindung der Formel

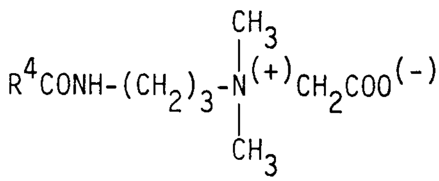

$R^4$ = Alkylrest der gehärteten Kokosfettsäure
verwendet. Die Zusammensetzung der Lösung beträgt:

| | |
|---|---|
| Betain | 43 Gew.-% |
| nichtionogenes Tensid | 2 bis 6 Gew.-% |
| Kaliumchlorid | 10 Gew.-% |
| Wasser | 45 bis 41 Gew.-% |

Tabelle II

| Gew.-% nichtionogenes Tensid* x = | Viskosität mPa.s bei 25 °C |
|---|---|
| 2 | Gel |
| 4 | 50 000 |
| 6 | 5 000 |

* Polyoxyethylenether von Monolaurinsäuresorbitanester mit 120 Oxyethyleneinheiten

Der Zusatz der nichtionogenen Tenside stört bei der weiteren Verwendung der Betainlösungen nicht, da die Betainlösungen bei ihrer Verwendung zur Herstellung von Shampoos oder Duschgelen im allgemeinen mit nichtionogenen Tensiden compoundiert werden. Der Zusatz von 3 bis 20 Gew.-% nichtionogener Tenside ist deshalb nur als Mindestmenge zu verstehen, um die gewünschte Viskositätserniedrigung sicherzustellen. Es ist selbstverständlich möglich, den Betainlösungen auch größere Mengen an nichtionogenen Tensiden zuzusetzen, wenn dies vom Verwendungszweck her betrachtet erwünscht ist.

Das erfindungsgemäße Verfahren gestattet es somit, flüssige, pump- und dosierfähige Betainlösungen, z.B. auf Basis von Kokosfettsäure, mit einem Betaingehalt von mindestens 37 Gew.-% bis etwa 75 Gew.-% herzustellen, wobei, wie bereits ausgeführt, die maximal erreichbare Konzentration insbesondere vom Rest $R^1$, der Menge des zugesetzten nichtionogenen Tensids und dem Gehalt an niederem Alkohol abhängt.

Die erfindungsgemäß hergestellten Lösungen lassen sich ohne weiteres verdünnen und bilden beim Verdünnen in der Regel keine Gelstrukturen. Die Betainlösungen können in üblicher Weise konfektioniert werden. Dabei soll unter dem Begriff Konfektionierung die Einstellung der gewünschten Konzentration, gegebenenfalls Zusatz von Farbstoffen, Parfümierungsmitteln, anderen hautpflegenden Substanzen und/oder Verdikkungsmitteln verstanden werden.

In den folgenden Beispielen werden das erfindungsgemäße Verfahren und der Effekt der Viskositätserniedrigung noch näher erläutert.

A. Herstellung wäßrig-alkoholischer Betainlösungen

Beispiel 1

Herstellung einer wäßrig-ethanolischen Lösung eines Betains der Formel

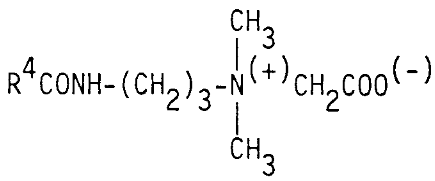

$R^4$ = Alkylrest einer gehärteten Kokosfettsäure

300 g eines Fettsäuredimethylaminopropylamids auf der Basis von gehärteter Kokosfettsäure werden mit 103 g Monochloressigsäure und 150 g Ethanol (96 %ig) in einen 1-l-Vierhalskolben, ausgerüstet mit Rührer, Thermometer, Rückflußkühler und Tropftrichter, eingewogen. Man heizt dieses Gemisch auf 80 °C auf, rührt dabei und läßt sodann unter Beibehaltung der Temperatur innerhalb von 3 Stunden 72 g KOH (86 %ig), gelöst in 55 g Wasser, zutropfen. Nach Zugabe der KOH läßt man das Reaktionsgemisch weitere 8 Stunden bei 80 °C unter Rühren reagieren. Danach wird das Reaktionsgemisch innerhalb von 1 Stunde auf Raumtemperatur abgekühlt. Durch Filtration werden 65 g ausgefallenes, verunreinigtes KCl abfiltriert. Der Filterrückstand enthält 52 g gereinigtes KCl. Dem Filtrat werden 35 g eines Polyoxyethylenglycerinmonorizinoleates mit 15 Oxyethyleneinheiten unter Rühren zugesetzt. Anschließend werden im Wasserbad bis maximal 100 °C 104 g Ethanol abdestilliert.

Es werden 541 g wäßrig-ethanolische Betainlösung mit folgender Zusammensetzung erhalten:

| | |
|---|---|
| Betain | 65 Gew.-% |
| KCl | 5,5 Gew.-% |
| Ethanol | 8 Gew.-% |
| nichtionogenes Tensid | 6,5 Gew.-% |
| Wasser | 15 Gew.-% |

Die Lösung ist schwach gelb gefärbt und homogen. Die Viskosität beträgt ca. 7 000 mPa.s bei 25 °C.

Beispiel 2

Herstellung einer wäßrig-ethanolischen Lösung eines Betains der Formel

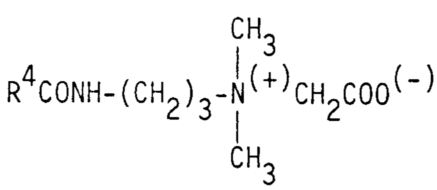

$R^4$ = Alkylrest einer $C_8$-Fettsäure

229 g Fettsäuredimethylaminopropylamid auf der Basis einer $C_8$-Fettsäure werden mit 103 g Monochloressigsäure und 150 g Ethanol (96 %ig) in einen 1-l-Vierhalskolben, ausgerüstet mit Rührer, Thermometer, Rückflußkühler und Tropftrichter eingewogen. Man heizt dieses Gemisch unter Rühren auf 80 °C auf und läßt sodann unter Beibehaltung der Temperatur innerhalb von 3 Stunden 71,6 g KOH (86 %ig), gelöst in 55 g Wasser, zutropfen. Man läßt das Reaktionsgemisch weitere 8 Stunden bei 80 °C reagieren. Danach wird das Reaktionsgemisch innerhalb 1 Stunde auf Raumtemperatur abgekühlt. Durch Filtration werden dann 83 g verunreinigtes KCl erhalten. Der Filterrückstand enthält 67 g gereinigtes KCl. Dem Filtrat werden 28,3 g eines Polyoxyethylenglycerinmonorizinoleates mit 15 Oxyethyleneinheiten zugesetzt. Anschließend werden

im Wasserbad bei maximal 100 °C 127 g Ethanol und 59 g Wasser abdestilliert.

Es werden 367 g wäßrig-ethanolische Betainlösung mit folgender Zusammensetzung erhalten:

| | |
|---|---|
| Betain | 75 Gew.-% |
| KCl | 4,1 Gew.-% |
| Ethanol | 6 Gew.-% |
| nichtionogenes Tensid | 7,6 Gew.-% |
| Wasser | 6,5 Gew.-% |

Das Produkt ist homogen, flüssig und hat eine Viskosität von ca. 20 000 mPa.s bei 25 °C.

Beispiel 3

Herstellung einer ethanolisch-wäßrigen Lösung eines Betains der Formel

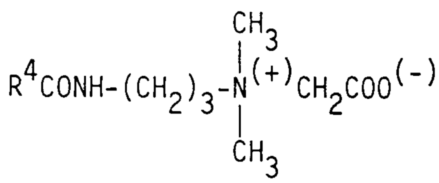

$R^4$ = Alkylrest einer gehärteten Kokosfettsäure

In einen 1-l-Vierhalskolben, bestückt mit Rührer, Thermometer, Rückflußkühler und Tropftrichter, werden 300 g Kokosfettsäuredimethylaminopropylamid, 103 g Monochloressigsäure und 74 g Ethanol (96 %ig) eingewogen. Unter Rühren läßt man bei 80 °C 73 g KOH (86 %ig), gelöst in 85 g Wasser, verteilt über 3 Stunden zutropfen. Man läßt ca. 8 Stunden nachreagieren, bis der Amidamingehalt < 2 % ist. Nach dem Abkühlen wird das ausgefallene KCl (= 54 g, entsprechend 65 % der Theorie) abfiltriert. Der Restgehalt KCl verbleibt im Filtrat gelöst. Dem Filtrat werden 45 g eines Polyoxyethylensorbitanmonostearates mit 80 Ethylenoxideinheiten zugesetzt.

Man erhält 614 g der gewünschten Lösung folgender Zusammensetzung:

| | |
|---|---|
| Betain | 57 Gew.-% |
| KCl | 4,7 Gew.-% |
| Ethanol | 12 Gew.-% |
| nichtionogenes Tensid | 7,3 Gew.-% |
| Wasser | 19 Gew.-% |

Die Viskosität der Lösung beträgt 300 mPa.s bei 25 °C.

B. Herstellung wäßriger Betainlösungen

Beispiel 4

Herstellung einer wäßrigen Lösung eines Betains der Formel

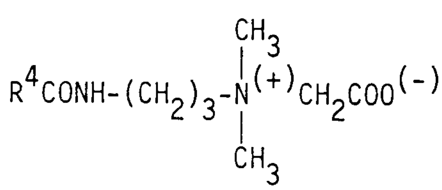

$R^4$ = Alkylrest einer gehärteten Kokosfettsäure
In einen 1-l-Vierhalskolben, ausgerüstet mit Rührer, Thermometer, Rückflußkühler und Tropftrichter, werden 104 g Monochloressigsäure und 315 g Wasser eingewogen. Man fügt unter Kühlung 72 g KOH (86 %ig) portionsweise unter Rühren zu.

Nach Zugabe von 41,5 g Polyoxyethylensorbitanmonolaurat mit 120 Oxyethyleneinheiten heizt man auf 80 °C auf und läßt über einen Zeitraum von 4 Stunden 300 g Kokosfettsäuredimethylaminopropylamid zutropfen. Nach weiteren 9 Stunden Reaktionszeit bei 80 °C liegt der Amidgehalt deutlich unter 2 %.

Es werden 825 g einer wäßrigen Betainlösung der folgenden Zusammensetzung erhalten:

| | |
|---|---|
| Betain | 43,4 Gew.-% |
| KCl | 10,3 Gew.-% |
| nichtionogenes Tensid | 5,0 Gew.-% |
| Wasser | 39,8 Gew.-% |

Die Lösung hat hellgelbe Farbe und eine Viskosität von ca. 6 500 mPa.s bei 25 °C.

Beispiel 5
(nichterfindungsgemäßer Vergleichsversuch)

Herstellung einer wäßrigen Lösung eines Betains der Formel

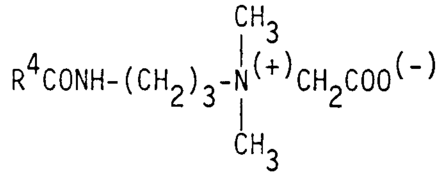

$R^4$ = Alkylrest einer gehärteten Kokosfettsäure
In einen 1-l-Vierhalskolben, ausgerüstet mit Rührer, Thermometer, Rückflußkühler und Tropftrichter, werden 128 g Natriumchloracetat, 290 g Kokosfettsäuredimethylaminopropylamid und 585 g Wasser unter Rühren auf 95 °C erwärmt und 1 Stunde bei dieser Temperatur gehalten. Sodann werden 3 g Natronlauge (50 %ig) zugegeben und das Reaktionsgemisch weitere 5 Stunden auf dieser Temperatur gehalten. Nach dem Abkühlen des Reaktionsgemisches erhält man als Endprodukt 990 g einer 36 %igen wäßrigen Lösung des gewünschten Betains mit beginnender Gelbildung und einer Viskosität von ca. 2 000 mPa.s bei 25 °C. Der Natriumchloridgehalt beträgt etwa 6,5 %.

Beispiel 6

Herstellung einer wäßrigen Lösung eines Betains der Formel

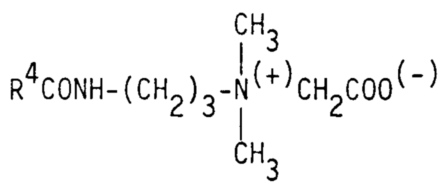

$R^4$ = Alkylrest einer $C_{10}$-Fettsäure
In einen 1-l-Vierhalskolben mit Rührer, Thermometer, Rückflußkühler und Tropftrichter werden 104 g Monochloressigsäure und 95 g Wasser eingewogen und unter Erwärmen auf 30 bis 40 °C gelöst. Danach werden vorsichtig 75 g KOH (86 %ig) zugegeben und gelöst. Nach der Neutralisation wird das Reaktionsgemisch auf 95 °C erwärmt. Man läßt 252 g Decylamidopropyldimethylamin in einem Zeitraum von 2,5

Stunden zutropfen, wobei die Temperatur auf 95 °C gehalten wird. Nach weiteren 2 Stunden bei 95 °C werden 34 g Polyoxyethylensorbitanmonolaurat mit 120 Oxyethyleneinheiten zugegeben. Das Gemisch wird weitere 2 Stunden bei 95 °C gerührt. Man erhält eine wäßrige Betainlösung, die bei Raumtemperatur flüssig ist und einen Betaingehalt von 57 Gew.-% aufweist.

Beispiel 7

Herstellung einer wäßrigen Losung eines Betains der Formel

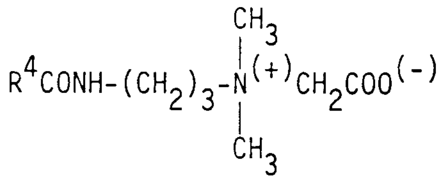

$R^4$ = Alkylrest einer $C_8$-Fettsäure
In einen 1-l-Vierhalskolben mit Rührer, Thermometer, Rückflußkühler und Tropftrichter werden 104 g Monochloressigsäure und 20 g Wasser eingewogen und unter Erwärmen auf 40 bis 50 °C gelöst. Danach werden vorsichtig 75 g KOH (86 %ig) zugegeben und gelöst. Anschließend wird das Reaktionsgemisch auf 95 °C erwärmt. Man läßt 228 g Octylamidopropyldimethylaminin einem Zeitraum von 2,5 Stunden zutropfen. Nach weiteren 2 Stunden bei 95 °C werden 27 g Polyoxyethylensorbitanmonolaurat mit 120 Oxyethyleneinheiten zugegeben. Man rührt weitere 2 Stunden bei 95 °C. Die erhaltene Betainlösung ist bei Raumtemperatur noch flüssig und gießfähig. Der Betaingehalt beträgt 65 %.

Beispiel 8

Herstellung einer wäßrigen Lösung eines Betains der Formel

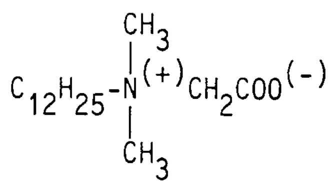

In einen 1-l-Vierhalskolben, ausgerüstet mit Rührer, Thermometer, Rückflußkühler und Tropftrichter, werden 104 g Monochloressigsäure, 92 g Wasser und 75 g KOH (86 %ig) eingewogen und unter Rühren und Kühlen umgesetzt. Sodann wird die Lösung auf 95 °C erwärmt und mit 220 g Dodecyldimethylamin über einen Zeitraum von 2,5 Stunden versetzt. Nach weiteren 2 Stunden Reaktionszeit bei 95 °C werden 55 g Polyoxyethylensorbitanmonolaurat mit 20 Oxyethyleneinheiten zugegeben. Das Reaktionsgemisch wird weitere 2 Stunden bei 95 °C belassen. Das nach dem Abkühlen erhaltene Produkt weist bei Raumtemperatur eine Viskosität von ca. 20 000 mPa.s bei 25 °C auf und ist gerade noch gießfähig. Die Ausbeute beträgt 530 g Betainlösung. Diese hat folgende Zusammensetzung:

| | |
|---|---|
| Dodecylbetain | 53 Gew.-% |
| KCl | 15,5 Gew.-% |
| nichtionogenes Tensid | 10,2 Gew.-% |
| Wasser | 20,8 Gew.-% |

Aus den Beispielen ergibt sich, daß durch den Zusatz der nichtionogenen Tenside die Viskosität der Betainlösungen wesentlich herabgesetzt wird:

Tabelle III

| $R^1$ | Gew.-% Betain ohne nichtionogenes Tensid (Vergleich) | Gew.-% Betain mit nichtionogenem Tensid (erfindungsgemäß) |
|---|---|---|
| $R^1 = R^4CONH(CH_2)_3$- <br> $R^4$ ist abgeleitet von: | | |
| $C_8$-Fettsäure | 46 20 000 mPa.s | 65 20 000 mPa.s |
| $C_{10}$-Fettsäure | 41 20 000 mPa.s | 57 20 000 mPa.s |
| Kokosfettsäure gehärtet | 36 20 000 mPa.s | 43,4 6 500 mPa.s |
| $R^1$ = Alkyl | | mit 10 Gew.-% nichtionogenem Tensid |
| $C_{12}H_{25}$- | 40 20 000 mPa.s | 53 20 000 mPa.s |
| | | mit 8 Gew.-% nichtionogenem Tensid |
| $C_{14}H_{29}$- | -- | 50 20 000 mPa.s |

**Patentansprüche**

**1.** Verfahren zur Herstellung von konzentrierten fließfähigen wäßrigen, gegebenenfalls niedere aliphatische Alkohole, vorzugsweise Ethanol, enthaltenden Lösungen von Betainen der allgemeinen Formel

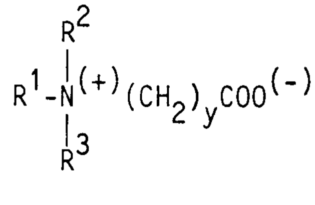

$R^1$ Alkylrest mit 6 bis 18 Kohlenstoffatomen oder der Rest $R^4CONH(CH_2)_x$-, wobei $R^4$ ein von einer Fettsäure mit 6 bis 18 Kohlenstoffatomen abgeleiteter Alkylrest und x = 2 oder 3 ist,

$R^2$, $R^3$ gleich oder ungleich, Alkylrest mit 1 bis 4 Kohlenstoffatomen,

x 2 oder 3,
y 1,2 oder 3,
durch Quaternierung eines tertiären Amins der allgemeinen Formel

$R^1-NR^2R^3$

mit ω-Halogenalkylcarbonsäuren $X(CH_2)_yCOOY$ oder deren Salzen (X = Halogenrest, Y = Wasserstoff-, Alkali- oder Ammoniumion) in wäßriger oder wäßrig-alkoholischer Lösung bei erhöhten Temperaturen, dadurch gekennzeichnet, daß man dem Reaktionsgemisch vor oder während der Quaternierungsreaktion oder der erhaltenen Lösung des Betains nichtionogene, wasserlösliche Tenside in solchen Mengen zusetzt, daß die fertige Lösung 3 bis 20 Gew.-% nichtionogene Tenside enthält.

**2.** Verfahren nach Anspruch 1, dadurch gekennzeichnet, daß man die nichtionogenen Tenside in solchen Mengen zusetzt, daß die fertige Lösung 3 bis 15 Gew.-% nichtionogene Tenside enthält.

**3.** Weitere Ausbildung des Verfahrens nach Anspruch 1, dadurch gekennzeichnet, daß man die Quaternierungsreaktion in wäßriger, mindestens 20 Gew.-% Ethanol enthaltender Lösung durchführt, das ausgefallene Salz nach der Quaternierungsreaktion abfiltriert und die gewünschte Konzentration des Betains in der Lösung durch Abdestillieren des Wassers bzw. des Wasser/Ethanolgemisches einstellt.

**4.** Verfahren nach Anspruch 1 oder 2, dadurch gekennzeichnet, daß man als wasserlösliche, nichtionogene Tenside Polyoxyethylenderivate von Fettalkoholen, Fettsäuren oder partiellen Fettsäureestern mehrwertiger Alkohole mit einem HLB-Wert von 14 bis < 20 zusetzt.

**5.** Verfahren nach Anspruch 4, dadurch gekennzeichnet, daß man als nichtionogene Tenside Polyoxyethylenester von Fettsäuren oder Polyoxyethylenether von partiellen Fettsäureestern des Glycerins oder des Sorbits, mit einem durchschnittlichen Gehalt von 10 bis 250 Oxyethyleneinheiten zusetzt.

## Claims

**1.** Process for the preparation of concentrated, flowable, aqueous solutions, optionally containing lower aliphatic alcohols, preferably ethanol, of betaines of the general formula

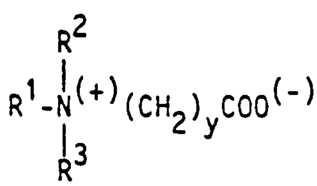

in which
$R^1$ is an alkyl radical having 6 to 18 carbon atoms or the radical $R^4CONH(CH_2)_x$-, where $R^4$ is an alkyl radical derived from a fatty acid having 6 to 18 carbon atoms and x is 2 or 3,
$R^2$ and $R^3$ are identical or different and are an alkyl radical having 1 to 4 carbon atoms,
x is 2 or 3,
y is 1, 2 or 3,
by quaternisation of a tertiary amine of the general formula

$R^1-NR^2R^3$

with ω-haloalkylcarboxylic acids $X(CH_2)_yCOOY$ or salts thereof (X = halogen radical, Y = hydrogen ion, alkali metal ion or ammonium ion) in aqueous or aqueous/alcoholic solution at increased temperatures, characterised in that non-ionic, water-soluble surfactants are added to the reaction mixture before or during the quaternisation reaction or to the resulting betaine solution, in such amounts that the finished solution contains 3 to 20 % by weight of non-ionic surfactants.

2. Process according to Claim 1, characterised in that the non-ionic surfactants are added in such amounts that the finished solution contains 3 to 15 % by weight of non-ionic surfactants.

3. Further embodiment of the process according to Claim 1, characterised in that the quaternisation reaction is carried out in aqueous solution containing at least 20 % by weight of ethanol, the salt which has precipitated after the quaternisation reaction is filtered off, and the desired betaine concentration in the solution is adjusted by distilling off the water or the water/ethanol mixture.

4. Process according to Claim 1 or 2, characterised in that the water-soluble, non-ionic surfactants employed are polyoxyethylene derivatives of fatty alcohols, fatty acids or partial fatty acid esters of polyhydric alcohols having an HLB value of 14 < 20.

5. Process according to Claim 4, characterised in that the non-ionic surfactants employed are polyoxyethylene esters of fatty acids or polyoxyethylene ethers of partial fatty acid esters of glycerol or of sorbitol, containing, on average, 10 to 250 oxyethylene units.

**Revendications**

1. Procédé de préparation de solutions de bétaïnes, concentrées, fluides, aqueuses, contenant éventuellement des alcools aliphatiques inférieurs, de préférence l'éthanol, répondant à la formule générale :

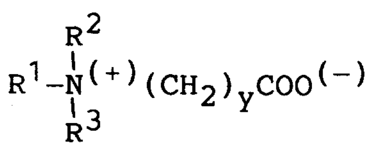

| | |
|---|---|
| $R^1$ | est un reste alkyle contenant de 6 à 18 atomes de carbone ou le reste $R^4CONH(CH_2)_x$-, où $R^4$ est un reste alkyle provenant d'un acide gras à 6 à 18 atomes de carbone et x = 2 ou 3, |
| $R^2$, $R^3$ | sont identiques ou différents et représentent un reste alkyle contenant de 1 à 4 atomes de carbone, |
| x | vaut 2 ou 3, |
| y | vaut 1, 2 ou 3, |

par quaternisation d'une amine tertiaire répondant à la formule générale :

$R^1$-$NR^2R^3$

avec des acides ω-halogénoalkylcarboxyliques $X(CH_2)_yCOOY$ ou leurs sels (x = reste halogène, Y = ion hydrogène, alcalin ou ammonium) en solution aqueuse ou aqueuse-alcoolique, à des températures augmentées, caractérisé en ce qu'on ajoute au mélange réactionnel, avant ou pendant la réaction de quaternisation, ou à la solution de bétaïne obtenue, des agents tensio-actifs non ionogènes solubles dans l'eau, en des quantités telles que la solution finale contient de 3 à 20 % en poids d'agents tensio-actifs non ionogènes.

2. Procédé selon la revendication 1, caractérisé en ce qu'on ajoute les agents tensio-actifs non ionogènes en des quantités telles que la solution finale contient de 3 à 15 % en poids d'agents tensio-actifs non ionogènes.

3. Perfectionnement du procédé selon la revendication 1, caractérisé en ce qu'on effectue la réaction de quaternisation dans une solution aqueuse contenant au moins 20 % en poids d'éthanol, on sépare par filtration le sel précipité après la réaction de quaternisation et on règle la solution à la concentration souhaitée de bétaïne en éliminant par distillation l'eau ou le mélange d'eau et d'éthanol.

4. Procédé selon la revendication 1 ou 2, caractérisé en ce qu'on ajoute, comme agents tensio-actifs non ionogènes solubles dans l'eau, des dérivés de polyoxyéthylène et d'alcools gras, d'acides gras ou d'esters partiels d'acides gras de polyols ayant un indice HLB de 14 à < 20.

**5.** Procédé selon la revendication 4, caractérisé en ce qu'on ajoute, comme agents tensio-actifs non ionogènes, des esters de polyoxyéthylène et d'acides gras ou des éthers de polyoxyéthylène et d'esters partiels d'acides gras du glycérol ou du sorbitol, contenant en moyenne de 10 à 250 unités d'oxyéthylène.